# EUROPEAN PATENT SPECIFICATION

(11) **EP 1 860 092 B1**
(45) Date of publication and mention of the grant of the patent: **11.01.2012**
(21) Application number: 06019095.6
(22) Date of filing: 12.09.2006
(51) Int. Cl.: C07C 51/265, C07C 63/38

(54) **Method for producing naphthalenedicarboxylic acid**
Verfahren zur Herstellung von Naphthalindicarbonsäure
Procédé de préparation d'acide 2,6-naphtalene dicarboxylique

(30) Priority: 22.05.2006 KR 20060045544
(43) Date of publication of application: 28.11.2007
(73) Proprietor: Hyosung Corporation, 431-080 Kyonggi-do (KR)
(72) Inventor: Chenon, Yang-Ho, Dongan-ku Anyang-si Kyonggi-do, 431-080 (KR); Choi, Young-Gyo, Dongan-ku Anyang-si Kyonggi-do, 431-080 (KR); Kwon, Ik-Hyun, Dongan-ku Anyang-si Kyonggi-do, 431-080 (KR)
(74) Representative: Hrovat, Andrea Darinka

(56) References cited:
- EP-A1- 0 999 199
- EP-B1- 0 872 470
- US-A- 5 183 933

## Description

### BACKGROUND OF THE INVENTION

### 1. Field of the Invention

The present invention relates to a method for producing naphthalenedicarboxylic acid (hereinafter naphthalene dicarboxylic acid is sometimes abbreviated to "NDA"), or "2,6-naphthalenedicarboxylic acid", and in particular, to a method for producing 2,6-NDA at low costs and a high efficiency, in which the amount of reactants used is reduced by recycling some of the product, mother liquor, and oxygen and a diluent gas of an oxidation reaction to the process, and the purity and yield of the product can be enhanced.

### 2. Description of the Related Art

It has been known that a 2,6-naphthalenedicarboxylic acid and ester thereof are each a compound of industrial importance as a starting raw material for a high performance polyester which is employed for the production of polyethylene naphthalate(PEN) in the form of fiber, film, bottles, engineering plastics and the like is excellent in tensile strength and heat resistance. The 2,6-NDA and an ester thereof that are used for such a purpose are required to be high pure.

Polyethylene naphthalate is produced by a process which comprises the steps of: producing naphthalenedicarboxylic acid by oxidizing 2,6-dimethylnaphthalene with molecular oxygen; producing naphthalene dicarboxylate by esterifying naphthalenedicarboxylic acid and purifying the product; and polymerizing the purified naphthalene dicarboxylate with ethylene glycol. In this production process, the process for oxidizing 2,6-dimethylnaphthalene requires a catalyst comprising cobalt, manganese and bromine.

In the process for producing polyethylene naphthalate, naphthalenedicarboxylic acid cannot be used directly for the polymerization process because a single oxidation reaction does not yield naphthalenedicarboxylic acid of high purity. The process for producing naphthalenedicarboxylic acid is associated with production of impurities such as 2,6-formylnaphthoic acid(2,6-FNA), naphthoic acid(NA), trimellitic acid(TMLA) and the like. Among the impurities, 2,6-formylnaphthoic acid(2,6-FNA) acts as a reaction terminator in the polyethylene naphthalate polymerization process, and when 2,6-FNA is present at a concentration above a certain level, polyethylene naphthalate having high molecular weight cannot be obtained. On the other hand, naphthoic acid(NA) is formed during a decomposition reaction of naphthalenedicarboxylic acid, and acts as a reaction terminator to lower the yield of the final product, naphthalenedicarboxylic acid. Trimellitic acid(TMLA) functions as a crosslinking agent in the process for polymerizing polyethylene naphthalate, and when TMLA is co-present with naphthalenedicarboxylic acid, it makes controlling of the properties of the final polymerization product, polyethylene naphthalate, difficult.

Therefore, there have been suggested a variety of methods for producing naphthalenedicarboxylic acid of high purity by reducing the amount of the impurities contained in naphthalenedicarboxylc acid. For example, US Patent No. 5,183,933 discloses a process for continuously oxidizing dimethylnaphthalene by using a catalyst comprising cobalt, manganese and bromine components. However, the naphthalenedicarboxylic acid obtained by the suggested process contains a large amount of colored impurities, and thus needs to be subjected to a complicated esterification process before being used in the polymerization of polyethylene naphthalate. Another US Patent No. 6,268,528 discloses a method for producing naphthalenedicarboxylic acid in a semi-continuous process by using a catalyst comprising cobalt, manganese and bromine components. According to the suggested method, a naphthalenedicarboxylic acid of relatively high purity can be obtained, but the purity of the obtained solid product reaches only 98.5 wt% at the maximum, and the solid product needs to be subjected to a separate purification process in order to be used for polymerization.

The 2,6-naphthalenedicarboxylic acids produced according to the prior arts or publicly known inventions do not have the purity sufficient for direct use in the polyethylene naphthalene polymerization process. Since the purity of 2,6-naphthalenedicarboxylic acid is relevant to the extent of impurities production, development of a production process which is capable of inhibiting impurities production is on demand. Such production of impurities is related to the type and amount of the catalyst used, reaction conditions for the oxidation reaction, the amount of controlling oxygen and a diluent gas, and the method of treating the reaction mother liquor. The present invention therefore suggests a method for producing 2,6-naphthalenedicarboxylic acid of high purity by appropriately controlling the aforementioned conditions.

### SUMMARY OF THE INVENTION

Accordingly, it is an object of the present invention to provide a method for producing 2,6-naphthalenedicarboxylic acid of high purity by controlling the conditions for oxidation reaction and recycling appropriate amount or some of diluent gas and by recycling an appropriate amount or some of reaction mother liquor.

Specifically, polyethylene naphthalate is produced by a process which comprises the steps of: producing naphthalenedicarboxylic acid by oxidizing 2,6-dimethylnaphthalene with molecular oxygen; producing naphthalene dicarboxylate by esterifying naphthalenedicarboxylic acid and purifying the product; and polymerizing the purified naphthalene dicarboxylate with ethylene glycol. In this production process, the process for oxidizing 2,6-dimethylnaphthalene requires a catalyst comprising cobalt, manganese and bromine.

According to an appropriate embodiment of the present invention, there is provided a method for producing naphthalenedicarboxylic acid comprising the steps of dissolving 2,6-dimethylnaphthalene in acetic acid solvent; oxidizing the dissolution product using oxygen and a diluent gas; crystallizing the naphthalenedicarboxylic acid which has been produced by oxidation; and separating the crystallized naphthalenedicarboxylic acid, wherein the amount of the diluent gas being discharged from and recycled to the oxidation process is controlled during the oxidation process, and the amount of the mother liquor being recycled to the dissolution process after crystallization is controlled during the separation process.

According to the invention, the oxygen content in the diluent gas being discharged is controlled to be 2 to 10%.

According to the invention, the amount of the mother liquor being recycled is controlled to be in the range of 5 to 20% by weight based on the acetic acid solvent.

According to another appropriate embodiment of the invention, the oxidation reaction is carried out at a temperature of 180 to 220°C and at a pressure of 15 to 30. kg/cm² by recycling the diluent gas.

According to another appropriate embodiment of the invention, the amounts of the diluent gas and the mother liquor that are recycled are controlled so that the product purity is not less than 99.3%.

According to another appropriate embodiment of the invention, the amounts of the diluent gas and the mother liquor that are recycled are controlled during the crystallization process so that the impurities include 25 ppm or less of trimellitic acid, and 40 ppm or less of 2,6-formylnaphthoic acid, naphthoic acid and methylnaphthalenecarboxylic acid.

According to another appropriate embodiment of the invention, the oxidation process is carried out in the presence of a complex catalyst containing cobalt, manganese and bromine components, and the amounts of the diluent gas and the mother liquor that are recycled are controlled during the oxidation process so that the weight ratio of cobalt atoms to 2,6-dimethylnaphthalene is 0.02 to 0.15, the weight ratio of manganese atoms to cobalt atoms is 0.05 to 1.0, and the weight ratio of bromine atoms to cobalt atoms is 0.8 to 2.0.

### BRIEF DESCRIPTION OF THE DRAWINGS

Fig. 1 is a schematic diagram of a process according to an embodiment of the present invention; and
Fig. 2 is a schematic diagram of the recycling process in a process for production of naphthalenedicarboxylic acid according to an embodiment of the invention.

### DESCRIPTION OF THE PREFERRED EMBODIMENTS

Hereinafter, the present invention will be described in detail with reference to Examples.

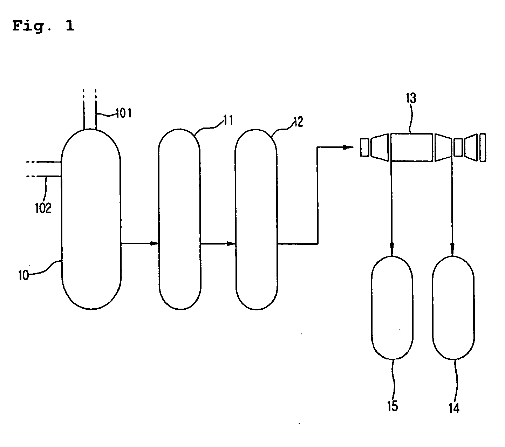

Fig. 1 is a schematic diagram of a process according to an embodiment of the invention.

The process for producing 2,6-naphthalenedicarboxylic acid comprises the steps of dissolving 2,6-dimethylnaphthalene in a solvent; producing 2,6-naphthalenedicarboxylic acid by oxidizing the dissolution product; crystallizing the produced 2,6-naphthalenedicarboxylic acid; and separating the crystallized 2,6-naphthalenedicarboxylic acid.

According to Fig. 1, 2,6-dimethylnaphthalene is introduced to a reactant preparation bath 10 through a first inlet 101, and at the same time, acetic acid and water as solvents are introduced to the reactant preparation bath 10 through a second inlet 102. The amount of the solvents can be determined by taking account of the solubility of 2,6-dimethylnaphthalene, but the amount is preferably 10- to 20-folds of the weight of 2,6-dimethylnaphthalene. The reaction in the reactant preparation bath 10 is intended to establish the conditions for an oxidation reaction. The product from the reactant preparation bath 10 with established conditions for the oxidation reaction is sent to an oxidation reactor 11, together with an oxygen-containing gas and catalyst. In the oxidation reactor 11, a reaction of oxidizing 2,6-dimethylnaphthalene by using molecular oxygen is carried out. The oxidation reaction may be carried out in the oxidation reactor at a temperature of 180 to 220°C and at a pressure of 15 to 30 kg/cm². When the reaction temperature is less than 180°C, the amounts of production of intermediate reaction products such as 2,6-formylnaphthoic acid and of by-products such as trimellitic acid, are increased. On the other hand, when the reaction temperature exceeds 220°C, the amount of trimellitic acid produced is not reduced, while the acetic acid solvent is reduced in a large amount by combustion. The suggested pressure provides a pressure range for maintaining the reactants in the liquid phase under the given temperature conditions.

The oxidation reaction is performed in the presence of catalyst. The catalyst used in the process of oxidizing 2,6-dimethylnaphthalene to 2,6-naphthalenedicarboxylic acid may be a complex catalyst system comprising transition metal catalyst components such as cobalt or manganese, and a bromine component in combination. The compound of cobalt component may be exemplified by cobalt acetate, cobalt naphthalate and cobalt carbonate, and the amount of the cobalt component compound may be, as expressed as a weight ratio of cobalt atoms to 2,6-dimethylnaphthalene, 0.02 to 0.15, preferably 0.04 to 0.12, and more preferably 0.06 to 0.1. The compound of manganese component may be exemplified by manganese acetate, manganese naphthalate, manganese carbonate and manganese bromide, and the weight ratio of manganese atoms to cobalt atoms may be 0.05 to 1.0, and preferably 0.15 to 0.4. The transition metal catalysts, namely, cobalt and manganese, can be used individually or in combination, but the total amount of the transition metal catalysts is, as expressed as a weight ratio of the components containing cobalt and manganese atoms to 2,6-dimethylnaphthalene, is 0.03 to 0.25, and preferably 0.05 to 0.2. This amount of catalyst is determined in relation to the amount of impurities produced. Specifically, when the amount of the transition metal catalysts is less than 0.03, conversion of 2,6-formylnaphthoic acid, which corresponds to an intermediate of the oxidation reaction, is difficult, and the yield of the desired final product is reduced. On the other hand, when the amount of the transition metal catalysts exceeds 0.25, the transition metal may form a complex with trimellitic acid that is present as an impurity, thus lowering the purity of 2,6-naphthalenedicarboxylic acid. The catalyst comprising a bromine component, which is to be combined with the transition metal catalysts, may be at least one selected from the group consisting of manganese bromide, cobalt bromide, sodium bromide, ammonium bromide and tetrabromoethane. The amount of the bromine component catalyst may be 0.8 to 2.0, and preferably 1.0 to 1.5, as the weight ratio of bromine atoms to cobalt atoms. When the amount of the bromine component is less than 0.8, the amounts of the transition metal complex and of 2,6-naphthalenedicarboxylic acid are increased. On the other hand, when the amount exceeds 2.0, the amount of the transition metal complex decreases, but the amount of the bromine compound increases, and thus the amount of colored impurities also increases. Such increase in impurities may lead to an increase in the throughput of the purification process.

The 2,6-naphthalenedicarboxylic acid produced by the oxidation reaction is crystallized in a crystallization bath 12. In the crystallization bath 12, a solid phase 2,6-naphthalenedicarboxylic acid is produced from the oxidation product, in the form of solids with a uniform size at normal pressure and at a temperature of 100 to 120°C. The crystallization bath 12 contains crystallized 2,6-naphthalenedicarboxylic acid as well as the reaction mother liquor containing organic materials and the catalyst. In order to separate 2,6-naphthalenedicarboxylic acid from the reaction mother liquor, the product from the crystallization bath 12 is transported to the solid-liquid separation apparatus 13, where the product is separated into solid components and liquid components. The solid-liquid separation apparatus 13 may be a known apparatus for separating solids from liquids by means of filtration, centrifugation or settling.

2,6-naphthalenedicarboxylic acid, which is a solid component separated from the solid-liquid separation apparatus 13, is transported to a solids reservoir 14 for storing the final product, and the reaction mother liquor containing various organic materials and catalyst is transported to a liquid reservoir 15. The liquid reservoir 15 is connected to the reactant preparation bath 10 so that a portion of the mother liquor may be recycled to the reactant preparation bath 10.

The process for production of 2,6-naphthalenedicarboxylic acid described above comprises an oxidation process. The oxidation process is meant by a process of oxidizing 2,6-dimethylnaphthalene, and this process is carried out by introducing a catalyst and oxygen-containing air. The oxygen-containing air should be discharged after the oxidation process, and the oxygen content in the gas being discharged should be limited to a certain range. When the oxygen concentration in the gas being discharged after the oxidation reaction exceeds 10%, there is a risk of explosion due to the acetic acid that is used as the solvent. Therefore, it is necessary to control the oxygen concentration in the gas being discharged, and accordingly, it is advantageous that a diluent gas is introduced together with oxygen to the oxidation process. This diluent gas may be introduced to the oxidation process through a recycling process. Furthermore, the mother liquor discharged from the crystallization process contains the transition metal catalyst used in the oxidation reaction. The transition metal catalyst in the mother liquor may be recovered through a separate process. However, such a separate process requires an additional increase in the production costs, and may bring a disadvantageous effect in the aspect that the entire production process should be performed in a continuous mode. Thus, in the process for production of 2,6-naphthalenedicarboxylic acid of the invention, the diluent gas used in the oxidation process and the mother liquor discharged from the crystallization process are recycled.

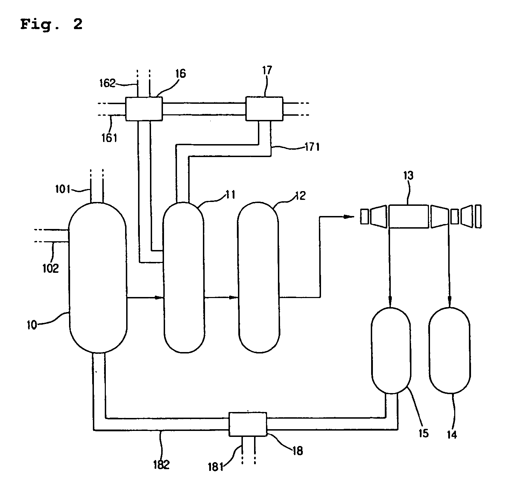

Fig. 2 is a schematic diagram of the recycling process in a process for production of 2,6-naphthalenedicarboxylic acid according to an embodiment of the invention.

According to Fig. 2, the diluent gas discharged from the oxidation reactor 11 and the mother liquor transported to the liquid reservoir 15 are recycled to the oxidation reactor 11 and the reactant preparation bath 10, respectively. Apparatuses other than the apparatus related to the recycling process in Fig. 2 have the same functions as those described for Fig. 1. In the following description, explanation on the apparatuses and reactions having the same functions will be omitted, and only the recycling process will be specifically explained.

Oxygen-containing air and the diluent gas containing nitrogen or carbon dioxide are introduced to the oxidation reactor 11 for the oxidation process, through an air inlet 161 and a gas inlet 162, respectively. The amounts of the gases introduced can be adjusted by an air flow meter 16 such that the pressure inside the reactor, that is, the pressure inside the oxidation reactor 11, is maintained at 15 to 30 kg/cm² as described above. Any residual gas remaining in the oxidation reactor 11 after the oxidation process is transported to a discharge flow meter 17 through an outlet 171. The discharge flow meter discharges some of the gas out of the system, while recycling the remaining portion of the gas to the oxidation reactor 11 via the air flow meter 16. The control of the amount discharged and the amount recycled is determined in accordance with the oxygen concentration in the discharged gas. For example, the amount of oxygen in the discharged gas should be maintained to be less than 10% based on the total gas weight. Therefore, when the amount of oxygen in the discharged gas is measured to be close to 10%, the ratio of recycling may be increased. When the ratio of recycling is increased, the oxygen content in the gas discharged through the outlet 171 can be reduced. On the other hand, a portion of the bromine component used as catalyst can be discharged through the discharged gas. If needed, the amount of the bromine component may be measured, and the measured amount can be used to control the amount of the bromine component to be introduced as catalyst. As shown in Fig. 2, the mother liquor transported to the liquid reservoir 15 is transported to a discharge controller 18. A portion of the mother liquor transported to the discharge controller 18, which contains 5 to 15% of water produced during the reaction, is discharged out of the system, while the remaining liquid is introduced to the reactant preparation bath 10. The control of the discharge amount and the recycling amount by the discharge controller 18 may be related to the purity of the 2,6-naphthalenedicarboxylic acid transported to the solids reservoir 14. The purity of the final product 2,6-naphthalenedicarboxylic acid that is transported to the solids reservoir 14 is at least 99.3%, according to the invention. Further, as described above, such purity is relevant to the amount of impurities contained in the mother liquor. If the purity value is close to 99.3%, the purity of the 2,6-naphthalenedicarboxylic acid produced thereafter needs to be further enhanced. Therefore, the recycling ratio of the mother liquor containing impurities needs to be reduced. However, even if the purity value is close to 99.9%, it is not necessary to increase the amount of the mother liquor being recycled to a level above what is needed. The amount of the mother liquor being recycled according to the invention is adjusted to the range of 5 to 20% by weight relative to the amount of the solvent introduced to the reactant preparation bath 10. This amount of the mother liquor being recycled can be determined by taking account of the recycling ratio of the catalyst. Substantially, when the recycling ratio of the mother liquor is 5 to 20% by weight relative to the amount of solvent, the recycling ratio of the catalyst contained in the mother liquor reaches 10 to 30% by weight relative to the amount of catalyst initially introduced, and thus the amount of fresh catalyst to be introduced can be effectively reduced.

The method for production according to the invention allows production of 2,6-naphthalenedicarboxylic acid having a high purity of 99.3%, preferably of 99.9%, by controlling the recycling ratio of the discharged gas or the recycling ratio of the mother liquor.

Now, specific examples for producing 2,6-naphthalenedicarboxylic acid according to the method of the invention will be described in the following.

### EXAMPLES

### EXAMPLE 1

A titanium reactor having a capacity of 300 L, which is equipped with a cooler, a heater and a stirrer, was charged with reactants and catalyst, and an oxidation reaction was performed. The oxidation reactor was set to a temperature of 200°C and a pressure of 20 kg/cm², and the stirrer was rotated at 700 rpm to disperse the reactant gases. During the reaction, air was introduced in an amount of about 35.7 moles per mole of 2,6-dimethylnaphthalene until the initial amount of the gas being recycled was secured, and was introduced in an amount of 22 moles per mole of 2,6-dimethylnaphthalene after the system was stabilized. As a diluent gas, nitrogen was introduced in an amount of 1.8 moles per mole of 2,6-dimethylnaphthalene, and after stabilization, the amount of the discharged gas to be recycled was adjusted such that the oxygen content in the discharged gas was 4 to 6% by weight. After the steps of oxidation reaction followed by crystallization and solid-liquid separation, the final product was obtained, and the final product was purged with BSTFA in a nitrogen environment and then subjected to analysis by gas chromatography. The amount of the mother liquor recycled during the production was adjusted to 10% by weight.

The amount of 2,6-dimethylnaphthalene used, the amount of acetic acid and the amount of catalyst are presented in Table 1, while the yield of the final product and the content of impurities are presented in Table 2.

### EXAMPLES 2 and 3

2,6-naphtheledicarboxylic acid was produced under the same conditions as in Example 1, except that the amounts of acetic acid and catalyst as well as the amount of the mother liquor that are recycled were varied. The compounds used for the reaction and the resulting products are presented in Table 1 and Table 2, respectively.

### COMPARATIVE EXAMPLES

### COMPARATIVE EXAMPLES 1 AND 2

The production was carried out under the same conditions as in Example 1, except that the discharged gas was not recycled in Comparative Example 1, while the oxygen content in the discharged gas was adjusted to 4 to 6% by weight, but the amount of the mother liquor being recycled was adjusted to the amount given in Table 1 in Comparative Example 2. The compounds used for the reaction and the resulting products are presented in Table 1 and Table 2, respectively.

**Table 1**

| | Example 1 | Example 2 | Example 3 | Comp. Ex. 1 | Comp. Ex. 2 |
|---|---|---|---|---|---|
| 2,6-Dimethylnaphthalene | 5 kg/hr | 5 kg/hr | 5 kg/hr | 5 kg/hr | 5 kg/hr |
| Acetic acid | 88 kg/hr | 84 kg/hr | 79 kg/hr | 69 kg/hr | 87 kg/hr |
| Cobalt | 6.21 wt% | 5.0 wt% | 5.0 wt% | 5.0 wt% | 5.0 wt% |
| Manganese | 1.58 wt% | 1.0 wt% | 1.0 wt% | 1.0 wt% | 1.0 wt% |
| Bromine | 3.87 wt% | 3.87 wt% | 3.87 wt% | 3.87 wt% | 3.87 wt% |
| Distilled water | 25.0 wt% | 23.0 wt% | 23.0 wt% | 23.0 wt% | 23.0 wt% |
| Amount of mother liquor recycled | 10 kg/hr | 5 kg/hr | 10 kg/hr | 20 kg/hr | 2 kg/hr |

| | | | | | |
|---|---|---|---|---|---|
| * The weight ratio given in Table 1 is a weight ratio relative to 100 wt% of dimethylnaphthalene. * The weight of cobalt, manganese and bromine in Table 1 is a weight of each atom, each atom is used as above mentioned the compounds, and each compound is added in accordance with the weight ratio of each atom. | | | | | |

**Table 2**

| | Example 1 | Example 2 | Example 3 | Comp. Ex. 1 | Comp. Ex. 2 |
|---|---|---|---|---|---|
| NDA | 99.41 wt% | 99.39 wt% | 99.35 wt% | 99.02 wt% | 99.26 wt% |
| TMLA | 19 ppm | 20 ppm | 23 ppm | 32 ppm | 27 ppm |
| FNA, NA, MNA | 40 ppm | 38 ppm | 41 ppm | 46 ppm | 43 ppm |
| Yield | 95.46% | 95.73% | 96.18% | 94.97% | 95.01% |

| | | | | | |
|---|---|---|---|---|---|
| * The yield given in Table 2 is a ratio comparing NDA with DMN (NDA/DMN) in molar units. * NDA: naphthalenedicarboxylic acid; TMLA: trimellitic acid; FNA: 2,6-formylnaphthoic acid; NA: naphthoic acid; MNA: methylnaphthalenecarboxylic acid; and DMN: 2,6-dimethylnaphthalene | | | | | |

According to Table 2, when the recycling ratio of the mother liquor is 5 to 20% by weight based on the mass of acetic acid introduced, the product can be obtained with high yield. When the reaction mother liquor is recycled in an excess amount as in Comparative Example 1, the purity and yield of the product are lowered. On the other hand, when the amount of the mother liquor being recycled is excessively small as in Comparative Example 2, the effect of recycling the mother liquor is hardly expected. Thus, it can be seen that when the oxidation reaction is carried out continuously, with the amount of the mother liquor being recycled being adjusted to 5 to 20% by weight based on the solvent, 2,6-naphthalenedicarboxylic acid of high purity and high chromaticity can be obtained. It was found that the effect of the amount of the discharged gas on the purity and yield was substantially negligible. However, the amount of gas being recycled may be related to the stability and economic aspects of the entire process.

The invention provides a method for producing 2,6-naphthalenedicarboxylic acid with stability and reduced production costs by controlling during the oxidation process so that a portion of the diluent gas being discharged is recycled. The method also allows production of 2,6-naphthalenedicarboxylic acid of high purity and high chromaticity by recycling a portion of the mother liquor.

## Claims

1. A method for producing 2,6-naphthalenedicarboxylic acid comprising the steps of:
dissolving 2,6-dimethylnaphthalene in acetic acid solvent;
oxidizing the dissolution product by using oxygen and a diluent gas;
crystallizing the 2,6-naphthalenedicarboxylic acid that has been produced by oxidation; and
separating the crystallized 2,6-naphthalenedicarboxylic acid,
wherein the amount of the diluent gas being discharged from and recycled to the oxidation is controlled during the oxidation process and wherein the oxygen content in the diluent gas being discharged is controlled to be 2 to 10% and the amount of the mother liquor being recycled to the dissolution process after crystallization is controlled to be 5 to 20% by weight based on the acetic acid solvent during the process for separation of 2,6-naphthalenedicarboxylic acid.

2. The method of claim 1, wherein the reaction in the oxidation process is carried out at a temperature of 180 to 220°C and at a pressure of 15 to 30 kg/cm² using recycled diluent gas.

3. The method of claim 1, wherein the amounts of the diluent gas and the mother liquor that are recycled are controlled to yield a product having a purity of not less than 99.3%.

4. The method of claim 1, wherein the amounts of the diluent gas and the mother liquor that are recycled are controlled so that the impurities include 25 ppm or less of trimellitic acid, and 40 ppm or less of 2,6-formylnaphthoic acid, naphthoic acid and methylnaphthalenecarboxylic acid.

5. The method of claim 1, wherein the oxidation process is carried out in the presence of a complex catalyst comprising cobalt, manganese and bromine components, and the amounts of the diluent gas and the mother liquor that are recycled are controlled so that the weight ratio of cobalt atoms to 2,6-dimethylnaphthalene is 0.02 to 0.15, the weight ratio of manganese atoms to cobalt atoms is 0.05 to 1.0, and the weight ratio of bromine atoms to cobalt atoms is 0.8 to 2.0.

## Patentansprüche

1. Verfahren zur Herstellung von 2,6-Naphthalendicarbonsäure, umfassend die Schritte:
Lösen von 2,6-Dimethylnaphthalen in Essigsäure-Lösungsmittel;
Oxidieren des Lösungsprodukts unter Verwendung von Sauerstoff und eines Diluent-Gases;
Kristallisieren der 2,6-Naphthalendicarbonsäure, welche durch Oxidation erhalten wurde; und
Abtrennen der kristallisierten 2,6-Naphthalendicarbonsäure;
wobei die Menge des Diluent-Gases, welches ausgetragen wird von der Oxidation und recycelt wird in die Oxidation, während dem Oxidationsverfahren gesteuert wird, und wobei der Sauerstoffgehalt in dem Diluent-Gas, welches ausgetragen wird, gesteuert wird, so dass er 2 bis 10 % beträgt, und die Menge der Mutterlauge, welche in das Lösungsverfahren nach Kristallisation recycelt wird, gesteuert wird, so dass sie 5 bis 20 Gew.-% bezogen auf das Essigsäure-Lösungsmittel beträgt, während dem Verfahren zur Abtrennung von 2,6-Naphtahlendicarbonsäure.

2. Verfahren nach Anspruch 1, wobei die Umsetzung in dem Oxidationsverfahren bei einer Temperatur von 180 bis 220°C und bei einem Druck von 15 bis 30 kg/cm² unter Verwendung von recyceltem Diluent-Gas durchgeführt wird.

3. Verfahren nach Anspruch 1, wobei die Mengen des Diluent-Gases und der Mutterlauge, welche recycelt werden, gesteuert werden, um ein Produkt mit einer Reinheit von nicht weniger als 99,3 % bereitzustellen.

4. Verfahren nach Anspruch 1, wobei die Mengen des Diluent-Gases und der Mutterlauge, welche recycelt werden, gesteuert werden, so dass die Verunreinigungen 25 ppm oder weniger Trimellithsäure und 40 ppm oder weniger 2,6-Formylnaphthoesäure, Naphthoesäure und Methylnaphthalencarbonsäure einschließen.

5. Verfahren nach Anspruch 1, wobei das Oxidationsverfahren in der Gegenwart eines Komplexkatalysators, umfassend Cobalt-, Mangan- und Bromkomponenten, durchgeführt wird und die Mengen des Diluent-Gases und der Mutterlauge, welche recycelt werden, gesteuert werden, so dass das Gewichtsverhältnis von Cobaltatomen zu 2,6-Dimethylnaphthalen 0,02 bis 0,15 beträgt, das Gewichtsverhältnis von Manganatomen zu Cobaltatomen 0,05 bis 1,0 beträgt und das Gewichtsverhältnis von Bromatomen zu Cobaltatomen 0,8 bis 2,0 beträgt.

## Revendications

1. Procédé de fabrication d'acide 2,6-naphtalènedicarboxylique comprenant les étapes consistant à :
- dissoudre le 2,6-diméthylnaphtalène dans un solvant d'acide acétique ;
- oxyder le produit de dissolution en utilisant de l'oxygène et un gaz diluant ;
- cristalliser l'acide 2,6-naphtalènedicarboxylique qui a été produit par oxydation ; et
- séparer l'acide 2,6-naphtalènedicarboxylique cristallisé,
dans lequel la quantité du gaz diluant libéré et recyclé vers l'oxydation est contrôlée pendant le procédé d'oxydation et dans lequel la teneur en oxygène dans le diluant libéré est contrôlée pour être de 2 à 10 % et la quantité de la liqueur mère recyclée vers le procédé de dissolution après la cristallisation est contrôlée pour être de 5 à 20 % en poids sur la base du solvant d'acide acétique pendant le procédé de séparation de l'acide 2,6-naphtalène-dicarboxylique.

2. Procédé selon la revendication 1, dans lequel la réaction dans le procédé d'oxydation est réalisée à une température de 180 à 220°C et à une pression de 15 à 30 kg/cm² en utilisant le gaz diluant recyclé.

3. Procédé selon la revendication 1, dans lequel les quantités du gaz diluant et de la liqueur mère qui sont recyclées sont ajustées pour donner un produit ayant une pureté de pas moins de 99,3 %.

4. Procédé selon la revendication 1, dans lequel les quantités du gaz diluant et de la liqueur mère qui sont recyclées sont contrôlées de sorte que les impuretés comprennent 25 ppm ou moins d'acide trimellitique et 40 ppm ou moins d'acide 2,6-formylnaphtoïque, d'acide naphtoïque et d'acide méthylnaphtalènecarboxylique.

5. Procédé selon la revendication 1, dans lequel le procédé d'oxydation est réalisé en présence d'un catalyseur complexe comprenant des composants à base de cobalt, de manganèse et de brome, et les quantités du gaz diluant et de la liqueur mère qui sont recyclées sont contrôlées de sorte que le rapport en poids des atomes de cobalt au 2,6-diméthylnaphtalène soit de 0,02 à 0,15, le rapport en poids des atomes de manganèse aux atomes de cobalt soit de 0,05 à 1,0, et le rapport en poids des atomes de brome aux atomes de cobalt soit de 0,8 à 2,0.
